# EUROPEAN PATENT APPLICATION

(11) **EP 1 930 075 A1**
(43) Date of publication of application: **11.06.2008**
(21) Application number: 06783182.6
(22) Date of filing: 05.09.2006
(51) Int. Cl.: B01J 23/62, B01J 27/18, B01J 37/16, C07C 29/14, C07C 33/02, C07B 61/00

(54) **HYDROGENATION CATALYST FOR CARBONYL GROUP, METHOD FOR PRODUCING SAME, AND METHOD FOR PRODUCING UNSATURATED ALCOHOL BY USING SUCH CATALYST**

(30) Priority: 07.09.2005 JP 2005259630
(71) Applicant: National University Corporation Nagaoka University, Nagaoka-shi, Niigata 940-2188 (JP)
(72) Inventor: INOUE, Yasunobu, Nagaoka-shi, Niigata 9402188 (JP); NISHIYAMA, Hiroshi, Nagaoka-shi, Niigata 9402188 (JP); SAITO, Nobuo, Nagaoka-shi, Niigata 9402188 (JP); TAKEUCHI, Junichi, Nagaoka-shi, Niigata 9402188 (JP)
(74) Representative: TBK-Patent
(86) International application number: PCT/JP2006/317493
(87) International publication number: WO 2007/029667

(57) **Abstract**

Provided are a hydrogenation catalyst for carbonyl groups which can produce an unsaturated alcohol by hydrogenating an unsaturated carbonyl compound with high selectivity by a simple process at low cost, a method of efficiently producing the hydrogenation catalyst, and a practical method of producing an unsaturated alcohol by using the hydrogenation catalyst. In the present invention, the hydrogenation catalyst is obtained by carrying a noble metal such as ruthenium as a catalyst component onto a carrier which is composed of an oxygen -containing gallium compound. Gallium oxyhydroxide, gallium oxide, gallium phosphate or the like can be used as the gallium compound, and a hydrogenation catalyst including the gallium compound carrier carrying 0.1 to 10% by weight of ruthenium is used suitably.

## Description

### Technical Field

The present invention relates to a hydrogenation catalyst for a carbonyl group, which includes a gallium compound carrier carrying a noble metal such as ruthenium (Ru) or platinum (Pt) thereon, a method of producing the catalyst, and a method of producing an unsaturated alcohol by selectively hydrogenizing an unsaturated carbonyl compound using the hydrogenation catalyst.

### Background Art

Unsaturated alcohols such as nerol and geraniol are important compounds as intermediates for the production of organic compounds useful as synthetic resins, drugs, flavors, and the like. The unsaturated alcohol is produced by hydrogenating a corresponding unsaturated carbonyl compound in the presence of a hydrogenation catalyst.

Conventionally, various hydrogenation catalysts to be used in the production of unsaturated alcohols have been known and examples thereof include a ruthenium/iron catalyst carried on carbon (see Patent Documents 1 and 2). In addition, a catalyst made of a ruthenium derivative associated with a water-soluble ligand or made of a complex salt of ruthenium with a water-soluble ligand has been proposed (see Patent Documents 3 and 4).
Patent Document 1: JP 58-27642 A
Patent Document 2: JP 2003-24555 A
Patent Document 3: JP 2520461 B
Patent Document 4: JP 2549158 B

However, any of prior arts of Patent Documents 1 and 2 makes it their essential features to use three components, a carbon carrier, ruthenium, andiron, which makes the production of catalysts complicated. Further, to improve rate of selective hydrogenation from an unsaturated carbonyl compound to an unsaturated alcohol, methanol and tri-methyl amine are added to a catalytic reaction system, in consequence there is a need of a post-treatment process of removing these components from a reaction product. Further, a reaction proceeds in a medium containing an organic solvent in the prior arts of Patent Documents 3 and 4, resulting in an increase in cost. Besides, there is a problem of requiring a distillation process for removal of an organic solvent.

Further, catalysts are known, in which each of catalyst carriers carries at least one metal selected from group VIII elements and at least one additional element M selected from the group consisting of germanium, tin, lead, rhenium, gallium, indium, gold, silver, and thallium (see Patent Document 5). However, the hydrogenation catalyst uses, as the essential feature thereof, three components including the catalyst carrier which makes the production of the catalyst complicated. Further, in this catalyst, the gallium used as an additional element M may be present in a metal state from a viewpoint of the production method thereof. Further, in performing selective hydrogenation from an unsaturated carbonyl compound to an unsaturated alcohol using the catalyst, the unsaturated carbonyl compound as a raw material is required to be diluted with a solvent such as n-heptane in order to raise the selectivity of the unsaturated alcohol. In this case, the removal of such an organic solvent requires a distillation process. Patent Document 5: US 6,294,696

### Disclosure of the Invention

### Problem to be solved by the Invention

Therefore, the present invention solves the problems of the conventional art as described above, and intends to provide a hydrogenation catalyst for a carbonyl group, which is capable of economically producing an unsaturated alcohol by hydrogenating an unsaturated carbonyl compound with high selectivity with a simple process and a method of efficiently producing the hydrogenation catalyst. Further, the present invention intends to provide a practical method of producing an unsaturated alcohol using the hydrogenation catalyst.

### Means for solving the Problems

As a result of intensive studies, the inventors of the present invention have completed the present invention by finding out that the above problems can be solved by preparing a hydrogenation catalyst including a carrier made of a gallium compound carrying a noble metal such as ruthenium or Pt thereon, as a catalyst component.
In other words, the present invention employs the following constructions 1 to 13:
1. A hydrogenation catalyst for a carbonyl group, comprising an oxygen-containing gallium compound carrier carrying a noble metal thereon.
2. A hydrogenation catalyst according to Item 1, wherein the oxygen-containing gallium compound is selected from gallium oxyhydroxide, gallium oxide, and gallium phosphate.
3. A hydrogenation catalyst according to Item 1 or 2, wherein the oxygen-containing gallium compound carrier carries 0.1 to 10% by weight of ruthenium thereon.
4. A hydrogenation catalyst according to Item 3, further comprising 0.1 to 10% by weight of platinum carried thereon.
5. A method of producing a hydrogenation catalyst for a carbonyl group compirising an oxygen-containing gallium compound carrier carrying a noble metal thereon, comprising the steps of:
   1) suspending the oxygen-containing gallium compound carrier in water;
   2) adding a noble metal salt solution as a catalyst active component to the suspension; and
   3) adding a water-soluble reductant to reduce the catalyst active component to deposit the catalyst active component on the carrier.
6. A method of producing the hydrogenation catalyst according to item 5, further comprising the steps of:
   4) separating the catalyst having the catalyst active component deposited on the carrier from an aqueous phase of the suspension of the carrier; and
   5) drying the catalyst which is separated.
7. A method of producing the hydrogenation catalyst according to Item 5 or 6, wherein the water-soluble reductant of the step 3) is selected from methanol, ethanol, formaldehyde, sodium phosphinate, dimethylamine-borane, sodium boronhydride, potassium boronhydride, lithium borohydride, lithium aluminum hydride, and hydrazine.
8. A method of producing the hydrogenation catalyst according to any one of Items 5 to 7, wherein the catalyst active component of the step 2) is a chloride, a nitrate, a nitrosyl nitrate, an oxide, a hydroxide, an acetylacetonate complex, a pipiridine complex, or an ammine complex of ruthenium.
9. A method of producing the hydrogenation catalyst according to Item 8, further comprising the steps of, after depositing ruthenium as a catalyst active component on a carrier in the step 3):
   3-1) resuspending the catalyst which is separated in water;
   3-2) adding a platinum salt solution to the suspension; and
   3-3) reducing the platinum salt by the addition of a water-soluble reductant to the suspension to cause further deposition of platinum on the catalyst.
10. A method of producing an unsaturated alcohol represented by the formula (2), comprising hydrogenating an unsaturated carbonyl compound represented by the following formula (1) in the presence of the hydrogenation catalyst according to any one of items 1 to 4: 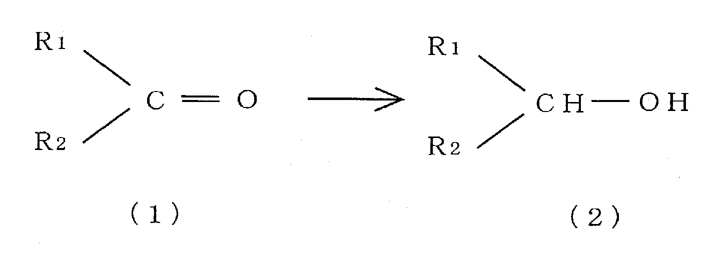 where: R₁ and R₂ are identical with or different from each other and each represent a hydrogen atom, a C1 to C10 saturated or unsaturated aliphatic group, a C1 to C10 saturated or unsaturated alicyclic group, or a C1 to C10 aromatic group; at least one of R₁ and R₂ contains an ethylenic double bond or a combination of R₁ and R₂ forms an ethylenic unsaturated alicyclic group; each of the aliphatic group, an alicyclic group, and an aromatic group may be substituted with one or two or more identical or different groups of a C1 to C4 alkyl group, a hydroxyl group, or a C1 to C4 alkoxy group.
11. A method of producing the unsaturated alcohol according to Item 10, wherein the carbonyl compound represented by the formula (1) includes an α,β-unsaturated carbonyl compound.
12. A method of producing the unsaturated alcohol according to Item 10 or 11, wherein the carbonyl compound represented by the formula (1) includes a citral.
13. A method of producing the unsaturated alcohol according to any one of Items 10 to 12, wherein the unsaturated carbonyl group is hydrogenated without dilution with a solvent.

### Effects of the Invention

The use of an oxygen-containing gallium compound as a carrier in a hydrogenation catalyst for carbonyl groups is newly proposed and exerts remarkable effects as follows.
1) The hydrogenation catalyst of the present invention principally includes two components: a carrier made of an oxygen-containing gallium compound; and ruthenium, so it can be easily produced at low cost.
2) The use of the novel catalyst of the present invention can lead to the production of an unsaturated alcohol by hydrogenation of an unsaturated carbonyl compound with high selectivity.
3) An unsaturated alcohol can be produced without using any solvent or auxiliary agent, so the process of alcohol production can be simplified and allows costs to be extensively reduced.

### Brief Description of Drawings

FIG. 1 is an electron micrograph of a gallium oxyhydroxide carrier obtained in Example 1.
FIG. 2 is an electron micrograph of a gallium oxide carrier obtained in Example 2.
FIG. 3 is an electron micrograph of a gallium phosphate carrier obtained in Example 3.

### Best Mode for carrying out the Invention

In the present invention, a hydrogenation catalyst for carbonyl groups is prepared by carrying a noble metal such as ruthenium as a catalyst component on an oxygen-containing gallium compound carrier. The amount of the catalyst component carried by the gallium compound is 0.1 to 10% by weight, specifically, preferably 1 to 3% by weight.

The oxygen-containing gallium compound used as a carrier is not specifically limited, but preferable gallium compounds include gallium oxyhydroxide, galliumoxide, and gallium phosphate. Any of those gallium compounds may be prepared by a routine method when a hydrogenation catalyst is produced or may be ones available in the market. Alternatively, a carrier may be used, which is prepared by coating a gallium compound on the surface of another carrier such as porous silica. The form and dimensions of the carrier are not specifically limited. In general, however, the carrier to be used may be in the form of a fine particle, a flake, or a porous body in a size of approximately 1 to 30 µm.
When ruthenium or the like is carried by metal gallium used as a carrier, gallium is molten under the hydrogenation conditions for a carbonyl compound because the metal gallium has a melting point of 29.8°C, and causes vigorous aggregation, thereby not functioning as a catalyst any more . The present invention overcomes these problems by using an oxygen-containing gallium compound as a carrier.

Hereinafter, the hydrogenation catalyst for carbonyl groups of the present invention will be described with reference to an example in which ruthenium is used as a catalyst active component.
The hydrogenation catalyst of the present invention can be produced by, for example, the following procedures:
1) suspending the oxygen-containing gallium compound carrier in water;
2) adding a noble metal salt solution as a catalyst active component to the suspension; and
3) adding a water-soluble reductant to reduce the catalyst active component to deposit the catalyst active component on the carrier.
   Instead of the step 3), there can also be employed the step 3') evaporating to dryness the carrier suspension added with the catalyst active component, sintering the dried product at 200 to 500°C in the air, followed by reducing in a stream of hydrogen at 200 to 600°C.

Further, in general, the following steps are employed after the above steps:
4) separating the catalyst having the catalyst active component deposited on the carrier from an aqueous phase of the suspension of the carrier; and
5) drying the catalyst which is separated.

The catalyst active component in the step 2) may be a chloride, a nitrate, a nitrosyl nitrate, an oxide, a hydroxide, an acetylacetonate complex, a pipiridine complex, or an ammine complex of ruthenium. Those catalyst components can be generally added to a carrier suspension as an aqueous solution. In addition, any of alkaline metal salts such as chlorides, nitrates, carbonates, and the like of lithium, sodium, potassium, rubidium, and cesium may be added together with the catalyst active component.

The water-soluble reductant in the above step 3 may be methanol, ethanol, formaldehyde, sodium phosphinate, dimethylamine-borane, sodium borohydride, potassium borohydride, lithium borohydride, lithium aluminum hydride, or hydrazine. Those reductants may be used alone or in combination of two or more.

After depositing ruthenium as a catalyst active component on a carrier in the step 3), the step 3-1) of resuspending the separated catalyst in water, the step 3-2) of adding a platinum salt solution to the suspension, and the step 3-3) of reducing the platinum salt by the addition of a water-soluble reductant to the suspension to cause further deposition of platinum on the catalyst, may be employed to produce a hydrogenation catalyst carrying ruthenium and platinum as catalyst components. Such a catalyst can exert higher catalyst activity.

Next, examples for producing the hydrogenation catalyst of the present invention using gallium oxide, gallium oxyhydroxide, and galliumphosphate as carriers will be further described in detail. However, the following specific examples do not restrict the present invention.

### (Production of ruthenium/gallium oxide catalyst)

Gallium nitrate is added to and dissolved in ethanol, and the pH thereof is increased by dropwisely adding an aqueous ammonium solution while stirring. The resulting solution is further stirred for 1 to 3 hours while being retained at a pH range of 5 to 6, thereby obtaining gel precipitate of gallium hydroxide. The resulting precipitate of gallium hydroxide is filtered by suction and then sintered at 500 to 800°C in the atmosphere, thereby obtaining a gallium oxide carrier.
Alternatively, commercially-available gallium oxide may be used as a carrier.

The gallium oxide carrier thus obtained is suspended in distilled water (step 1) and added with ruthenium in the form of a metal salt solution as an active component, followed by stirring for 30 minutes to 1 hour (step 2). Then, the temperature of the suspension is kept at room temperature to 70°C and gradually added with a water-soluble reductant to simultaneously carry out the carrying and reduction of ruthenium as an active component (step 3) .
Subsequently, the suspension is filtered by suction and a ruthenium/gallium oxide catalyst is separated from an aqueous phase (step 4), and then washed with isopropyl alcohol or ethanol, followed by drying at room temperature in the atmosphere (step 5).
In the step 2), an alkali metal salt and a lanthanoid metal salt may be added independently or simultaneously with each other.
Further, as an alternative method of the aforementioned method using the reduction of a liquid-phase, an applicable method includes allowing a carrier suspension added with a catalyst active component to evaporation to dryness, sintering the component in the air at a temperature of 200 to 500°C, and then reducing the component in the gas flow of hydrogen at 200 to 600°C.

### (Production of ruthenium/gallium phosphate catalyst)

Gallium nitrate is dissolved in distilled water and the solution is added with phosphoric acid and stirred. The solution is dropwisely added with an aqueous ammonium solution to increase the pH thereof and then stirred for 1 to 3 hours at a pH range of 4 to 6, thereby obtaining white precipitate. The precipitate is filtered by suction, dried at 100 to 200°C, and sintered at 800 to 1200°C in the atmosphere, thereby obtaining a gallium phosphate carrier.

The gallium carrier thus obtained is suspended in distilled water (step 1) and added with ruthenium in the form of a metal salt solution as an active component, followed by stirring for 30 minutes to 1 hour (step 2). Then, the temperature of the suspension is kept at room temperature to 70°C and gradually added with a water-soluble reductant to simultaneously carry out the carrying and reduction of ruthenium as an active component (step 3).
Subsequently, the suspension is filtered by suction and a ruthenium/gallium phosphate catalyst is then separated from an aqueous phase (step 4) and washed with isopropyl alcohol or ethanol, followed by drying at room temperature in the atmosphere (step 5).

In the step 2), an alkali metal salt and a lanthanoid metal salt may be added independently or simultaneously with each other.
Further, as an alternative method of the aforementionedmethod using the reduction of a liquid-phase, an applicable method includes allowing a carrier suspension added with a catalyst active component to evaporation to dryness, sintering the component in the air at a temperature of 200 to 500°C, and then reducing the component in the gas flow of hydrogen at 200 to 600°C.

### (Production of ruthenium/gallium oxyhydroxide catalyst)

An aqueous gallium nitrate solution is added with ammonia, urea, or hexamethylene tetramine and stirred overnight at a liquid temperature of 20 to 50°C. Further, the solution is stirred for additional two hours at a liquid temperature of 70 to 90°C, thereby obtaining white precipitate. The precipitate is cooled and filtered, washed with isopropyl alcohol or ethanol, and then dried at room temperature to 350°C, thereby obtaining a gallium oxyhydroxide carrier.
Further, gallium nitrate is pulverized with a mortar and sintered at a temperature range of 200 to 400°C for 5 to 20 hours in the atmosphere, thereby obtaining δ-gallium oxide. The δ-gallium oxide is mixed with distilled water, and hydrothermal synthesis is carried out in an autoclave at a temperature range of 150 to 300°C for 24 to 48 hours, thereby obtaining a gallium oxyhydroxide carrier.
In addition, gallium oxyhydroxide available in the market may be used as a carrier.

The gallium oxyhydroxide carrier is suspended in distilled water (step 1) and then added with ruthenium as an active component in the form of a metal salt solution, followed by stirring for 30 minutes to 1 hour (step 2). Then, the temperature of the suspension is kept at room temperature to 70°C and gradually added with a water-soluble reductant to simultaneously carry out the carrying and reduction of ruthenium as an active component (step 3) .
Subsequently, the suspension is filtered by suction and a ruthenium/gallium oxyhydroxide catalyst is separated from an aqueous phase (step 4) and then washed with isopropyl alcohol or ethanol, followed by drying at room temperature in the atmosphere (step 5).

In the step 2), an alkali metal salt and a lanthanoid metal salt may be added independently or simultaneously with each other.
Further, as an alternative method of the aforementionedmethod using the reduction of a liquid-phase, an applicable method includes allowing a carrier suspension added with a catalyst active component to evaporation to dryness, sintering the component in the air at a temperature of 200 to 500°C, and then reducing the component in the gas flow of hydrogen at 200 to 600°C.
The hydrogenation catalyst of the present invention principally includes two components: a carrier made of an oxygen-containing gallium compound; and ruthenium, so it can be easily produced at low cost.

Byusing the hydrogenation catalyst for a carbonyl group according to the present invention, an unsaturated carbonyl compound represented by the following formula (1) is selectively hydrogenated. Thus, an unsaturated alcohol represented by the formula (2) can be effectively produced.

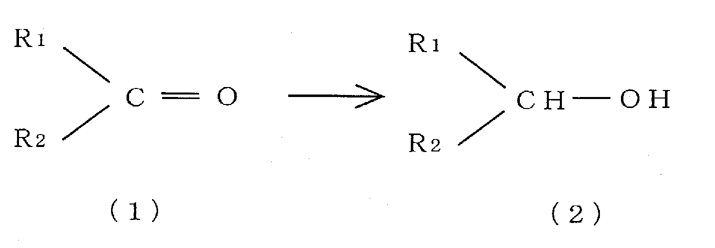

where: R₁ and R₂ are identical with or different from each other and each represent a hydrogen atom, a C1 to C10 saturated or unsaturated aliphatic group, a C1 to C10 saturated or unsaturated alicyclic group, or a C1 to C10 aromatic group; at least one of R₁ and R₂ contains an ethylenic double bond or a combination of R₁ and R₂ forms an ethylenic unsaturated alicyclic group; each of the aliphatic group, an alicyclic group, and an aromatic group may be substituted with one or two or more identical or different groups of a C1 to C4 alkyl group, a hydroxyl group, or a C1 to C4 alkoxy group.

Specific examples of R₁ and R₂ include: hydrogen; methyl, ethyl, propyl, isopropyl, n-butyl, i-butyl, t-butyl, pentyl, hexyl, heptenyl, octyl, nonyl, and decyl; 1-propenyl, 2-propenyl, 2-methyl-2-propenyl, 1-pentenyl, 1-methyl-2-pentenyl, isopropenyl, 1-butenyl, hexenyl, octenyl, and nonenyl or decenyl; and benzyl and phenyl or naphthyl. Each of those examples may be substituted with one or two or more identical or different groups of a C1 to C4 alkyl group, a hydroxyl group, or a C1 to C4 alkoxy group.

Examples of preferable unsaturated carbonyl compound represented by the formula (1) include citronellal, H-geranylacetone, H-nerolidol, methylvinyl ketone, mesityl oxide, pseudoionone, dihydrofarnesyl acetone, lysmeral, and methyl hexenone. An example of a particularly preferable unsaturated carbonyl compound includes citronellal or α,β-unsaturated carbonyl compound such as acrolein, methacrolein, crotonaldehyde, prenal, farnesal, or citral. Of those, citral is more preferable.

Citrals include citral A (trans form) represented by the following formula (3) and citral B (cis form) represented by the formula (4). When the carbonyl group is selectivelyhydrogenated, geraniol of interest represented by the formula (5) and nerol of interest represented by the formula (6) are generated. In addition, citronellal of the formula (7), citronellol of the formula (8), tetrahydrogeraniol of the formula (9), or the like may also be generated as a by-product.

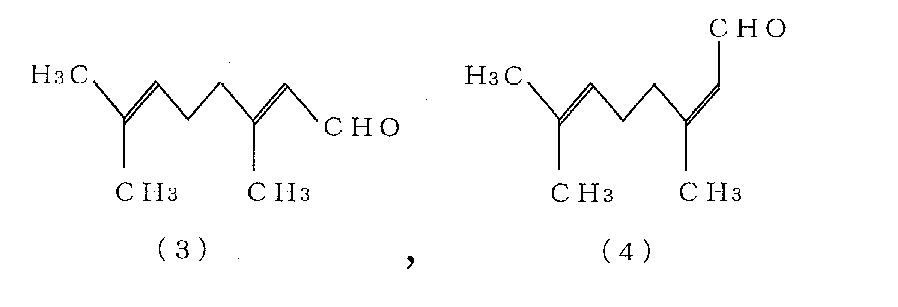

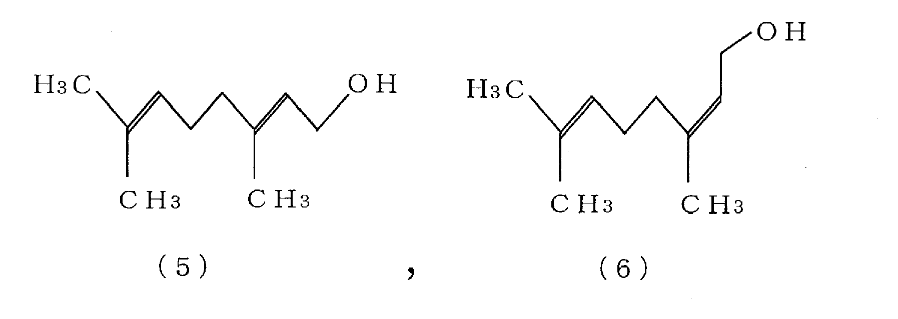

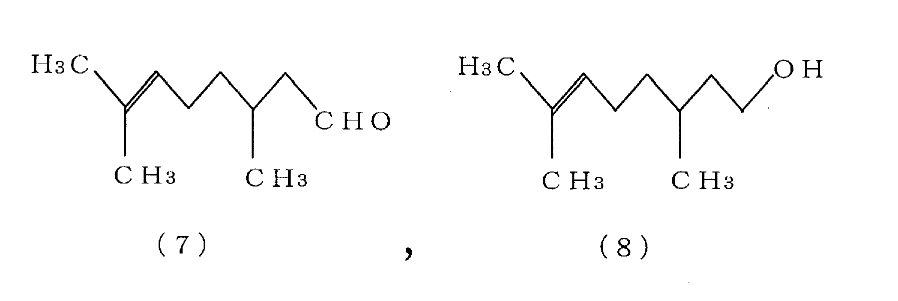

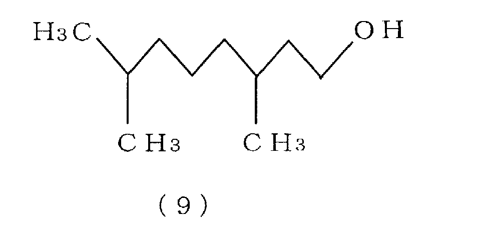

In the case of using the hydrogenation catalyst of the present invention, only an aldehyde group can be hydrogenated with high selectivity without using a solvent for diluting a raw material and an additive such as trimethylamine which are required for the conventional hydrogenation catalyst. In addition, geraniol or nerol of interest can be obtained in good yield while preventing a by-product from being generated. Therefore, the separation and purification of a product of interest can be easily carried out. Therefore, it becomes possible to extensively reduce the production costs of the product.

### Examples

Hereinafter, the present invention will be further described with reference to examples. However, those specific examples are not provided for limiting the present invention.

### (Example 1: Production of ruthenium/gallium oxyhydroxide catalyst)

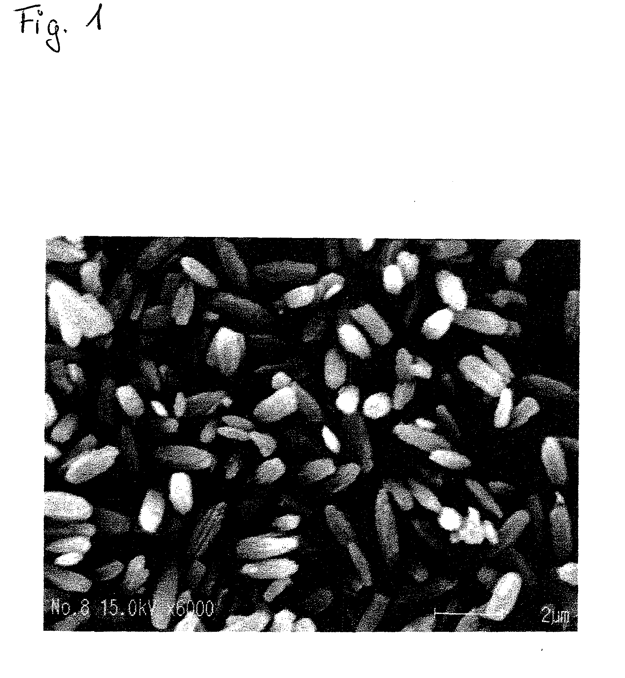

A 1-L separable flask was added with 500 ml of distilled water to dissolve 13.64 g of gallium nitrate. The solution was added with 70.13 g of hexamethylenetetramine and stirred at room temperature for 12 hours, followed by further stirring at 90°C for 2 hours. The solution was cooled and the resulting precipitate was then filtered by suction. The precipitate was washed with isopropyl alcohol and then dried at 300°C in the atmosphere, thereby obtaining a gallium oxyhydroxide carrier. FIG. 1 represents an electron micrograph of the gallium oxyhydroxide carrier.
The gallium oxyhydroxide in an amount of 2.0 g was suspended in 200 ml of distilled water and added with 0.1314 g of ruthenium chloride. Subsequently, a solution prepared by dissolving 2 g of sodium borohydride in 50 ml of distilled water was gradually dropped into the suspension and stirred for 2 hours to carry out liquid-phase reduction, thereby performing the carrying of 2.5% by weight of ruthenium on the carrier.
Subsequently, the catalyst suspension carrying ruthenium was filtered by suction. Then, the catalyst was washed with distilled water and ethanol, and dried at room temperature in the atmosphere, thereby obtaining a ruthenium/gallium oxyhydroxide catalyst.

### (Example 2: Production of ruthenium/gallium oxide catalyst)

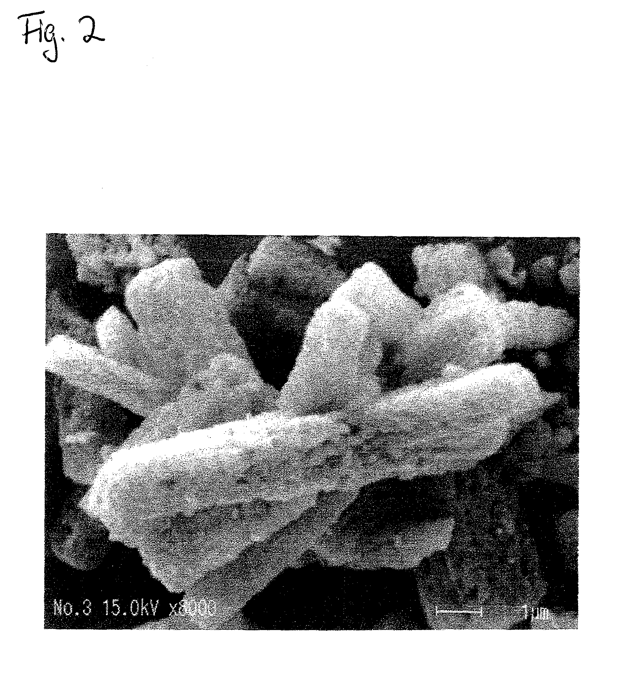

Ethanol in an amount of 200 ml was added to a 500-ml beaker to dissolve 13.7 g of gallium nitrate. The solution was dropwisely added with an aqueous ammonia solution to increase the pH of the solution to 5.2. The solution was stirred at room temperature for 2 hours, thereby obtaining the precipitate of gelled gallium hydroxide. The precipitate thus obtained was filtered by suction and then sintered at 800°C in the atmosphere, thereby obtaining a gallium oxide carrier. FIG. 2 represents an electron micrograph of the resulting gallium oxide carrier.
The gallium oxide in an amount of 1.5 g was suspended in 30 ml of ethanol and added with 0.148 g of a ruthenium acetylacetonate complex, followed by stirring at 60°C for 3 hours. The suspension was evaporated to dryness and then heated at 150°C in the air, followed by a reduction treatment at 400°C in the gas flow of hydrogen. Consequently, a ruthenium/gallium oxide catalyst carrying 2.5% by weight of ruthenium was obtained.

### (Example 3: Production of ruthenium/gallium phosphate catalyst)

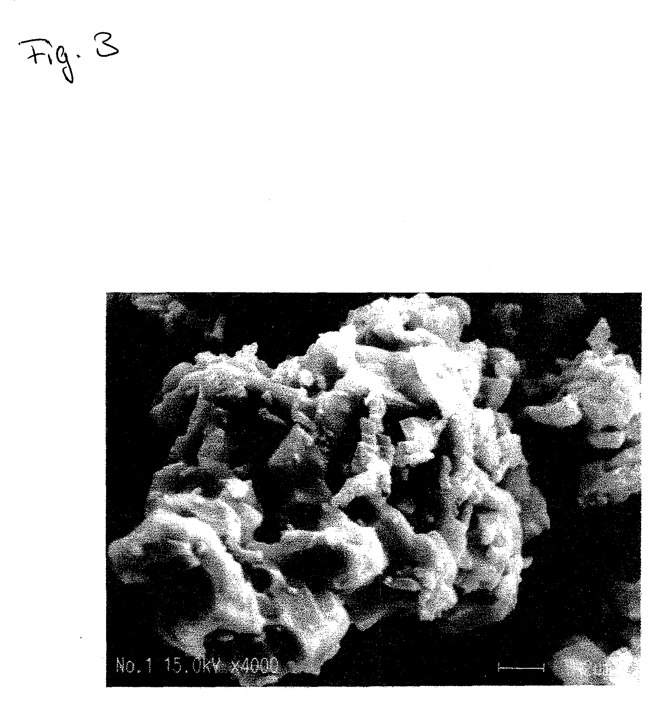

Distilled water in an amount of 200 ml was added to a 500-ml beaker to dissolve 15. 5 g of gallium nitrate. The solution was added with 4.8 g of phosphoric acid and stirred. The solution was dropwisely added with an aqueous ammonia solution to increase the pH of the solution to 5.0, and the solution was stirred for 1 hour, thereby obtaining white precipitate. The precipitate was filtered by suction, heated at 160°C for 2 hours in the atmosphere, and then sintered at 1, 000°C in the atmosphere, thereby obtaining a gallium phosphate carrier. FIG. 3 represents an electron micrograph of the resulting gallium phosphate carrier.
The gallium phosphate in an amount of 1.5 g was suspended in 30 ml of ethanol and added with 0.0986 g of a ruthenium chloride and 0.026 g of rubidium nitrate, followed by stirring at 60°C for 3 hours. The suspension was evaporated to dryness and then heated at 150°C for 1 hour in the air, followed by a reduction treatment at 400°C in the gas flow of hydrogen. Consequently, a ruthenium/gallium phosphate catalyst carrying 2.5% by weight of ruthenium was obtained.

### (Example 4: Selective hydrogenation of citral)

2 g of catalyst powder obtained in Example 1 was introduced into an autoclave of 200 ml in volume and then added with 130 ml of citral. After sealing the autoclave, nitrogen gas was repeatedly introduced into and discharged from the autoclave 3 times at a pressure of 1 MPa while stirring. Subsequently, the nitrogen gas was substituted with hydrogen gas at a pressure of 1.3 MPa and then heated up to 120°C. During the hydrogenation, samples were taken from a reaction vessel at regular intervals and analyzed by gas chromatography.
The conversion rate of citral, the selectivity of nerol/geraniol generated on the basis of such a conversion rate, and the by-products are listed in Table 1.

**Table 1**

| Citral conversion rate (%) | Selectivity of product (%) | | | | |
|---|---|---|---|---|---|
| | Nerol/ger aniol | Citronellal | Citronellol | Tetrahydro geraniol - | Unknown substance |
| 11.35 | 100.00 | 0.00 | 0.00 | 0.00 | 0.00 |
| 35.44 | 97.52 | 0.00 | 0.00 | 0.00 | 2.48 |
| 55.32 | 96.80 | 0.00 | 0.86 | 0.00 | 2.34 |
| 60.87 | 96.90 | 0.00 | 0.87 | 0.00 | 2.23 |
| 84.72 | 96.44 | 0.00 | 1.40 | 0.00 | 2.16 |

### (Example 5)

1.5 g of catalyst powder obtained in Example 2 was introduced into an autoclave of 100 ml in volume and then added with 65 ml of citral. After sealing the autoclave, nitrogen gas was repeatedly introduced into and discharged from the autoclave 3 times at a pressure of 1 MPa while stirring. Subsequently, the nitrogen gas was substituted with hydrogen gas at a pressure of 1.3 MPa and then heated up to 120°C. During the hydrogenation, samples were taken from a reaction vessel at regular intervals and analyzed by gas chromatography.
The conversion rate of citral, the selectivity of nerol/geraniol generated on the basis of such a conversion rate, and the by-products are listed in Table 2.

**Table 2**

| Citral conversion rate (%) | Selectivity of product (%) | | | | |
|---|---|---|---|---|---|
| | Nerol/ger aniol | Citronellal | Citronellol | Tetrahydro geraniol | Unknown substance |
| 9.87 | 81.16 | 0.00 | 0.00 | 0.00 | 18.84 |
| 22.67 | 83.98 | 0.00 | 2.15 | 0.00 | 13.88 |
| 42.17 | 87.02 | 0.00 | 1.47 | 1.17 | 10.33 |
| 69.97 | 89.01 | 0.00 | 1.18 | 1.29 | 8.53 |

### (Example 6)

1.5 g of catalyst powder obtained in Example 3 was introduced into an autoclave of 100 ml in volume and then added with 65 ml of citral. After sealing the autoclave, nitrogen gas was repeatedly introduced into and discharged from the autoclave 3 times at a pressure of 1 MPa while stirring. Subsequently, the nitrogen gas was substituted with hydrogen gas at a pressure of 1.3 MPa and then heated up to 120°C. During the hydrogenation, samples were taken from a reaction vessel at regular intervals and analyzed by gas chromatography.
The conversion rate of citral, the selectivity of nerol/geraniol generated on the basis of such a conversion rate, and the by-products are listed in Table 3.

**Table 3**

| Citral conversion rate (%) | Selectivity of product (%) | | | | |
|---|---|---|---|---|---|
| | Nerol/ger aniol | Citronellal | Citronellol | Tetrahydro geraniol | Unknown substance |
| 3.52 | 97.9 | 0.00 | 0.00 | 0.00 | 2.1 |
| 9.57 | 100.0 | 0.00 | 0.00 | 0.00 | 0.0 |
| 22.39 | 95.9 | 0.00 | 0.00 | 0.00 | 4.1 |
| 36.29 | 95.9 | 0.00 | 0.00 | 0.00 | 4.1 |

### (Example 7)

2.0 g of gallium oxyhydroxide prepared by the procedures of Example 1 was suspended in 200 ml of distilled water. Then, 0.134 g of ruthenium chloride was added to the suspension and stirred. Subsequently, a solution prepared by dissolving 2 g of sodium borohydride in 50 ml of distilled water was slowly dropped into the suspension and was stirred for 2 hours to carry out liquid-phase reduction, thereby 2.5% by weight of ruthenium was carried on the carrier. The catalyst suspended solution carrying ruthenium was filtered by suction. Then, the catalyst was washed with distilled water and ethanol. The catalyst was resuspended in 200 ml of distilled water and 0.133 g of chloroplatinate (IV) hexahydrate was then dissolved in the suspended solution. Subsequently, a solution prepared by dissolving 2 g of sodium borohydride in 50 ml of distilled water was slowly dropped to the suspension and was stirred for 2 hours to carry out liquid-phase reduction, thereby the gallium oxyhydroxide catalyst carrying 2.5% by weight of ruthenium further carried 2 . 5% by weight of platinum. The catalyst suspended was filtered by suction and the catalyst was then washed with distilled water and ethanol, followed by drying in the air. Consequently, a catalyst containing ruthenium and platinum as catalyst components was obtained.

### (Example 8)

2 g of catalyst powder obtained in Example 7 was introduced into an autoclave of 200 ml in volume and then added with 130 ml of citral. After sealing the autoclave, nitrogen gas was repeatedly introduced into and discharged from the autoclave 3 times at a pressure of 1 MPa while stirring. Subsequently, the nitrogen gas was substituted with hydrogen gas at a pressure of 1.3 MPa and then heated up to 120°C. During the hydrogenation, samples were taken from a reaction vessel at regular intervals and analyzed by gas chromatography.
The conversion rate of citral, the selectivity of nerol/geraniol generated on the basis of such a conversion rate, and the by-products are listed in Table 4.

**Table 4**

| Reaction time (h) | Citral conversion rate (%) | Selectivity of product (%) | | | |
|---|---|---|---|---|---|
| | | Nerol/ger aniol | Citronellal | Citronellol | Unknown substance |
| 1 | 21.2 | 98.51 | ND | ND | 1.49 |
| 3 | 62.6 | 97.70 | ND | 0.80 | 1.90 |
| 3.5 | 81.5 | 97.23 | ND | 0.88 | 1.89 |
| 4 | 91.9 | 96.97 | ND | 1.05 | 1.97 |

In the above description, the hydrogenation catalyst for carbonyl groups, in which a gallium compound carrier carries ruthenium, has been described. In the present invention, the catalyst active component which can be used may be a noble metal, such as Pt, Rh, or Ir, Co, or the like.
For instance, when Pt is used as a catalyst active component, the conversion rate of citral is decreased in comparison with that of ruthenium for the same reaction time, but the selectivity of nerol/geraniol becomes 100%. Therefore, depending on the application, any of other noble metals may be selected as a catalyst active component.

### (Example 9: Production of Pt/gallium oxyhydroxide catalyst)

2.0 g of gallium oxyhydroxide was suspended in 200 ml of distilled water. Then, 0.133 g of chloroplatinate was added to the suspension and stirred. Subsequently, a solution prepared by dissolving 2 g of sodium borohydride in 50 ml of distilled water was slowly dropped into the suspension and was stirred for 2 hours to carry out liquid-phase reduction, thereby the carrying of 2.5% by weight of Pt was performed.
Subsequently, the catalyst suspension carrying Pt was filtered by suction. Then, the catalyst was washed with distilled water and ethanol, followed by drying in the air. Consequently, a Pt/gallium oxyhydroxide catalyst was obtained.
The catalyst active component which can be used may be ammonium platinous chloride or ammonium platinic chloride instead of chloroplatinate.

### (Example 10)

2 g of Catalyst powder obtained in Example 9 was introduced into an autoclave of 200 ml in volume and then added with 130 ml of citral. After sealing the autoclave, nitrogen gas was repeatedly introduced into and discharged from the autoclave 3 times at a pressure of 1 MPa while stirring. Subsequently, the nitrogen gas was substituted with hydrogen gas at a pressure of 1.3 MPa and then heated up to 120°C. During the hydrogenation, samples were taken from a reaction vessel at regular intervals and analyzed by gas chromatography.
In a reaction time of 6 hours, the conversion rate of citral was 9.8% and the selectivity of nerol/geraniol was 100%.

## Claims

1. A hydrogenation catalyst for a carbonyl group, comprising an oxygen-containing gallium compound carrier carrying a noble metal thereon.

2. A hydrogenation catalyst according to claim 1, comprising an oxygen-containing gallium compound selected from gallium oxyhydroxide, gallium oxide, and gallium phosphate.

3. A hydrogenation catalyst according to claim 1 or 2, wherein
the oxygen-containing gallium compound carrier carries 0.1 to 10% by weight of ruthenium thereon.

4. A hydrogenation catalyst according to claim 3, further comprising 0.1 to 10% by weight of platinum carried thereon.

5. A method of producing a hydrogenation catalyst for a carbonyl group comprising an oxygen-containing gallium compound carrier carrying a noble metal thereon, comprising the steps of:
1) suspending the oxygen-containing gallium compound carrier in water;
2) adding a noble metal salt solution as a catalyst active component to the suspension; and
3) adding a water-soluble reductant to reduce the catalyst active component to deposit the catalyst active component on the carrier.

6. A method of producing the hydrogenation catalyst according to claim 5, further comprising the steps of:
4) separating the catalyst having the catalyst active component deposited on the carrier from an aqueous phase of the suspension of the carrier; and
5) drying the catalyst which is separated.

7. A method of producing the hydrogenation catalyst according to claim 5 or 6, wherein
the water-soluble reductant of the step 3) is selected from methanol, ethanol, formaldehyde, sodium phosphinate, dimethylamine-borane, sodium boronhydride, potassium boronhydride, lithium borohydride, lithium aluminum hydride, and hydrazine.

8. A method of producing the hydrogenation catalyst according to any one of claims 5 to 7, wherein
the catalyst active component of the step 2) is a chloride, a nitrate, a nitrosyl nitrate, an oxide, a hydroxide, an acetylacetonate complex, a pipiridine complex, or an ammine complex of ruthenium.

9. A method of producing the hydrogenation catalyst according to claim 8, further comprising the steps of, after depositing ruthenium as a catalyst active component on a carrier in the step 3) :
3-1) resuspending the catalyst which is separated in water;
3-2) adding a platinum salt solution to the suspension; and
3-3) reducing the platinum salt by the addition of a water-soluble reductant to the suspension to cause further deposition of platinum on the catalyst.

10. A method of producing an unsaturated alcohol represented by the formula (2), comprising hydrogenating an unsaturated carbonyl compound represented by the following formula (1) in the presence of the hydrogenation catalyst according to any one of claims 1 to 4: 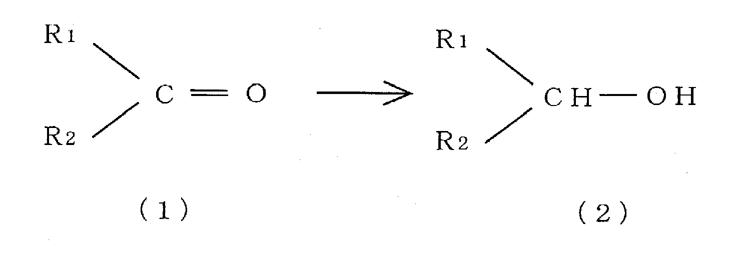 where: R₁ and R₂ are identical with or different from each other and each represent a hydrogen atom, a C1 to C10 saturated or unsaturated aliphatic group, a C1 to C10 saturated or unsaturated alicyclic group, or a C1 to C10 aromatic group; at least one of R₁ and R₂ contains an ethylenic double bond or a combination of R₁ and R₂ forms an ethylenic unsaturated alicyclic group; each of the aliphatic group, an alicyclic group, and an aromatic group may be substituted with one or two or more identical or different groups of a C1 to C4 alkyl group, a hydroxyl group, or a C1 to C4 alkoxy group.

11. A method of producing the unsaturated alcohol according to claim 10, wherein
the carbonyl compound represented by the formula (1) includes an α,β-unsaturated carbonyl compound.

12. A method of producing the unsaturated alcohol according to claim 10 or 11, wherein
the carbonyl compound represented by the formula (1) includes a citral.

13. A method of producing the unsaturated alcohol according to any one of claims 10 to 12, wherein
the unsaturated carbonyl group is hydrogenated without dilution with a solvent.
